# EUROPEAN PATENT APPLICATION

(11) **EP 2 853 259 A1**
(43) Date of publication of application: **01.04.2015**
(21) Application number: 13186700.4
(22) Date of filing: 30.09.2013
(51) Int. Cl.: A61K 9/127

(54) **Reconstituted high density lipoproteins composition and uses thereof**

(71) Applicant: Université Pierre et Marie Curie (Paris 6), 75005 Paris (FR)
(72) Inventor: Kontush, Anatol, 75018 Paris (FR); Chapman, John, 94100 Saint-Maur-des-Fossés (FR); Lhomme, Marie, 78320 Le Mesnil-Saint-Denis (FR)
(74) Representative: Icosa

(57) **Abstract**

The present invention relates to a reconstituted high density lipoprotein composition comprising negatively charged phospholipids to enhance cholesterol clearance, reduce inflammation and other anti-atherosclerotic actions.

## Description

### FIELD OF INVENTION

The present invention relates to a reconstituted high density lipoprotein (HDL) composition to enhance cholesterol clearance, reduce inflammation and other anti-atherosclerotic actions.

### BACKGROUND OF INVENTION

Low circulating levels of high density lipoprotein (HDL) represent a strong, independent risk factor for premature atherosclerosis and coronary heart disease (CHD). Low HDL-cholesterol (HDL-C) states (<40 mg/dl) are the most common form of dyslipidemia in CHD subjects. Dyslipidemias with low HDL-C and ApoA-I (Apolipoprotein A-I) levels are characteristic of metabolic disease associated with high cardiovascular (CV) risk, such as Type 2 diabetes and Metabolic Syndrome (MetS), and involve perturbations of HDL metabolism.

Epidemiological and clinical studies have established an inverse association between levels of high-density lipoprotein cholesterol (HDL-C) and risk of CV disease (reviewed in Assmann G et al., 2004, Circulation 109(23 Suppl 1):III8-14). More particularly, clinical administration of reconstituted HDL compositions has shown to confer beneficial effects to hypercholesterolemic subjects suffering from recent acute coronary syndromes (ACS).

Typically, such reconstituted HDL compositions comprise a protein such as Apo-AI, a lipid such as phosphatidylcholine and a detergent such as cholate or deoxycholate. In addition, cholesterol may be included. As discussed in US patent 5,652,339, it may be advantageous to produce reconstituted HDL compositions without using organic solvents, which in some cases are used for dissolving the lipid component (e.g. phosphatidylcholine) when producing the reconstituted HDL composition. A reconstituted HDL composition of this type, designated CSL-111, was clinically trialed but the higher dosage treatment was discontinued early following liver function test abnormalities. Subjects treated with CSL-111 showed beneficial trends in indices of plaque burden. However, statistical significance was not obtained in percentage change in atheroma volume or nominal change in plaque volume when compared with placebo (Tardif JC et al., 2007, JAMA-Exp. 297(15):1675-82). Recently, patent application WO2012/000048 disclosed the necessity to reduce detergent within the formulation in order to reduce hepatic toxicity.

However, there is a need to improve reconstituted HDL compositions to obtain a significant effect for a treatment of a disorder associated to an HDL dysfunction metabolism, an inflammatory disorder or a CV disorder without strong side-effects. The Applicant believes that understanding HDL function and properties in order to target these disorders is a necessity to develop new drugs but also to unravel new biomarkers specific to these disorders.

An original and innovative LC/MS/MS (Liquid chromatography-tandem mass spectrometry) approach was developed to determine the lipidome (phospholipid and sphingolipid profile) of a subject. This technology revealed marked heterogeneity in the lipidome composition across human plasma HDL subpopulations associated with multiple biological functions of HDL, and notably cholesterol efflux capacity and antioxidative, anti-thrombotic, anti-inflammatory and anti-apoptotic activities. Therefore, the present invention provides new reconstituted HDL compositions and their use for treating a disorder related to a dysfunction of HDL metabolism an inflammatory disorder or a CV disorder.

### SUMMARY

One object of the invention is a reconstituted high density lipoprotein composition comprising apolipoprotein, fragments or combinations thereof and at least one negatively charged phospholipid, fragments or combinations thereof.

In one embodiment of the invention said reconstituted high density lipoprotein is a small, dense HDL particle.

In another embodiment of the invention said small, dense HDL particle has a density between 1.12 and 1.25 g/mL, preferably 1.18 and 1.25 g/mL.

In another embodiment of the invention said reconstituted high density lipoprotein has a size between 6 to 12 nm, preferably 8 to 10 nm, most preferably 8 to 9 nm.

In another embodiment of the invention the negatively charged phospholipid is selected from the groups of: phosphatidyl serine, phosphatidyl inositol, phosphatidyl glycerol, most preferably phosphatidyl serine.

In another embodiment of the invention, said reconstituted high density lipoprotein further comprises at least one zwitterionic phospholipid.

In another embodiment of the invention said zwitterionic phospholipid is phosphatidyl choline (PC).

In another embodiment of the invention said reconstituted high density lipoprotein further comprises a detergent.

In another embodiment of the invention said reconstituted high density lipoprotein further comprises a stabilizer.

Another object of the invention is a reconstituted high density lipoprotein composition for use in the treatment of a disorder related to the dysfunction of HDL metabolism.

Another object of the invention is a reconstituted high density lipoprotein composition for use in the treatment of an inflammatory disorder.

Another object of the invention is a reconstituted high density lipoprotein composition for use in the treatment of a cardiovascular disorder.

### DEFINITIONS

In the present invention, the following terms have the following meanings:
- **"Treat, treating**, **treatment":** "Treating" or "treatment" or "alleviation" refers to both therapeutic treatment and prophylactic or preventative measures; wherein the object is to prevent or slow down the targeted pathologic condition or disorder. Those in need of treatment include those already with the disorder as well as those prone to have the disorder or those in whom the disorder is to be prevented. A subject or mammal is successfully "treated" for a disease if, after receiving the treatment according to the present invention, the subject or mammal shows observable and/or measurable reduction in or absence of one or more of the following: reduction in the number of pathogenic cells; reduction in the percent of total cells that are pathogenic; and/or relief to some extent, one or more of the symptoms associated with the specific disease or condition; reduced morbidity and mortality, and improvement in quality of life issues. The above parameters for assessing successful treatment and improvement in the disease are readily measurable by routine procedures familiar to a physician.
- **"Therapeutically effective amount"** refers to the level or amount of agent that is aimed at, without causing significant negative or adverse side effects to the target, (1) delaying or preventing the onset of a disorder related to dysfunction of HDL metabolism, an inflammatory disorder and/or a CV disorder; (2) slowing down or stopping the progression, aggravation, or deterioration of one or more symptoms of a disorder related to dysfunction of HDL metabolism, an inflammatory disorder and/or a CV disorder; (3) bringing about ameliorations of the symptoms of a disorder related to dysfunction of HDL metabolism, an inflammatory disorder and/or a CV disorder; (4) reducing the severity or incidence of a disorder related to dysfunction of HDL metabolism, an inflammatory disorder and/or a CV disorder; or (5) curing a disorder related to dysfunction of HDL metabolism, an inflammatory disorder and/or a CV disorder. An effective amount may be administered prior to the onset of a disorder related to dysfunction of HDL metabolism, an inflammatory disorder, and/or a CV disorder for a prophylactic or preventive action. Alternatively or additionally, the effective amount may be administered after initiation of a disorder related to dysfunction of HDL metabolism, an inflammatory disorder and/or a CV disorder, for a therapeutic action.
- **"Subject"** refers to a mammal, preferably a human. In one embodiment the subject is a female. In another embodiment the subject is a male.
- **"Pharmaceutically acceptable"** refers to compounds and compositions which may be administered to mammals without undue toxicity. Accordingly, a **"Pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, and general safety and purity standards as required by FDA Office of Biologics standards.
- **"Biological sample"** refers to a sample from the subject. Examples of biological sample include, but are not limited to, blood, plasma, serum, saliva, lymph, ascetic fluid, cystic fluid, urine, bile, nipple exudate, synovial fluid, bronchoalveolar lavage fluid, sputum, amniotic fluid, chorionic villi, peritoneal fluid, cerebrospinal fluid, pleural fluid, pericardial fluid, semen, saliva, sweat and alveolar macrophages. Preferably, said biological sample is plasma or serum.
- **"Reconstituted high density lipoprotein (HDL)"** refers to i) artificially-produced HDL composition, chemical synthetic HDL or recombinant HDL composition that is functionally similar to, analogous to, corresponds to, or mimics HDL typically present in blood plasma. Reconstituted HDL compositions include within their scope "HDL mimetics" and "synthetic HDL particles", and ii) modified naturally occurring HDL.
- **"Apolipoprotein fragments"** refer to cellular components, metabolites, secreted molecules and compounds resulting from the metabolism of apolipoproteins. Fragments may be obtained, for example, by recovering the supernatant of a culture of apolipoproteins or by extracting cell components or cell fractions, metabolites or secreted compounds from a culture of apolipoproteins. The term fragment may also refer to a degradation product. A fragment may correspond to a component in the isolated form or to any combination of one or more components derived from apolipoproteins.
- **"Inflammatory disorder"** refers to disorders associated with inflammation. Inflammation can be a part of the complex biological response of tissues to harmful stimuli, such as pathogens, damaged cells, toxins, or chemical irritants. Inflammation can also be due to other causative agents such as hormonal imbalance or autoimmune reactions. The inflammatory process involves complex biological cascades of molecular and cellular signals that alter physiological responses. Inflammatory disorders involve the local vascular system or/and the immune system. In some embodiments, the inflammatory disorder can be considered as an "acute inflammatory disorder". For example, an acute inflammatory disorder can be observed the days following a trauma or an infection. Frequently, during an acute inflammatory disorder the liver synthesizes acute phase proteins or acute phase reactants that are detectable in the blood stream. In addition, during an acute inflammatory episode, local inflammatory cells, e.g., neutrophils and macrophages secrete a number of cytokines into the bloodstream, most notably IL-1, IL-6, IL-11, and TNF-alpha. In some embodiments the inflammatory disorder can be considered as a "chronic inflammatory disorder". Frequently, a chronic inflammatory disorder is an inflammatory disorder that does not resolve after a period of weeks, months or longer. For example, a chronic inflammatory disorder can be due to hormonal imbalance or autoimmune reactions and are frequently intertwined with various internal and external factors that promote and protect against the said disorder. Inflammatory disorders are frequently associated with reduced circulating levels and diminished anti-atherogenic function of HDL.

### DETAILED DESCRIPTION

One object of the invention is a composition of reconstituted high density lipoprotein (rHDL) comprising Apolipoprotein or fragments or combinations thereof and at least one negatively charged phospholipid or fragments or combinations thereof.

In one embodiment of the invention, the rHDL composition is an artificially-produced HDL composition.

In another embodiment of the invention, the rHDL composition is a naturally occurring HDL composition that is modified by enrichment in negatively charged phospholipid or fragments or combinations thereof.

In one embodiment of the invention, the rHDL of the invention is a small dense HDL particle, which is well known in the art.

In one embodiment of the invention, the rHDL of the invention has a density between 1.12 and 1.25 g/mL, preferably 1.18 and 1.25 g/mL.

In one embodiment of the invention, the reconstituted HDL of the invention has a size of 6 to 12 nm, preferably 8 to 10 nm, most preferably 8 to 9 nm.

In one embodiment of the invention, the reconstituted HDL of the invention is a flattened discoidal particle.

In another embodiment of the invention, the reconstituted HDL of the invention is a spherical particle.

In another embodiment of the invention, the reconstituted HDL of the invention are a mixture of flattened discoidal particles and spherical particles.

In one embodiment, the apolipoprotein of the composition is Apolipoprotein A-I (ApoA-I).

The nature of the apolipoproteins comprising the apolipoprotein fraction of the reconstituted HDL particle is important but not critical for success. Virtually any apolipoprotein and/or fragments or analog thereof that provides therapeutic and/or prophylactic benefit as described herein can be included in the charged complexes. Moreover, any alpha-helical peptide or peptide analog, or any other type of molecule that "mimics" the activity of an apolipoprotein (such as, for example ApoA-I) in that it can activate the Lecithin-cholesterol acyltransferase (LCAT) or form discoidal particles when associated with lipids, can comprise the charged complexes, and is therefore included within the definition of "apolipoprotein". Examples of suitable apolipoproteins include, but are not limited to, preproapolipoprotein forms of ApoA-I; pro- and mature forms of human ApoA-I, ApoA-I Milano; and active polymorphic forms, isoforms, variants and mutants as well as truncated forms, the most common of which are ApoA-IM (APOA-IM) and ApoA-Ip (ApoA-Ip). Apolipoproteins mutants containing cysteine residues are also known, and can also be used (see, e.g., U.S. 2003/0181372). The apolipoproteins may be in the form of monomers or dimers, which may be homodimers or heterodimers. For example, homo- and heterodimers (where feasible) of pro- and mature ApoA-I (Duverger et al., 1996, Arterioscler. Thromb. Vase. Biol. 16(12):1424-29), A[rho]oA-IM (Franceschini et al., 1985, J. Biol. Chem. 260:1632-35), ApoA-IP (Daum et al., 1999, J. MoI. Med. 77:614-22). The apolipoproteins may include residues corresponding to elements that facilitate their isolation, such as His tags, or other elements designed for other purposes, so long as the apolipoprotein retains some biological activity when included in a complex.

Such apolipoproteins can be purified from animal sources (and in particular from human sources) or produced recombinantly as it is well-known in the art, see e.g., Chung et al., 1980, J. Lipid Res. 21(3):284-91; Cheung et al., 1987, J. Lipid Res. 28(8):913-29. See also U.S. Patent Nos. 5,059,528; 5,128,318; 6,617,134; and U.S. Publication Nos. 2002/0156007, 2004/0077541, and 2004/0266660.

Non-limiting examples of peptides and peptide analogs that correspond to apolipoproteins, as well as agonists that mimic the activity of ApoA-I, ApoA-IM, that are suitable for use as apolipoproteins in the charged complexes and compositions described herein are disclosed in U.S. Pat. Nos. 6,004,925; 6,037,323; and 6,046,166, U.S. Pat. No. 5,840,688; U.S. publications 2004/0266671, 2004/0254120, 2003/0171277 and 2003/0045460, and U.S. publication 2003/0087819, the disclosures of which are incorporated herein by reference in their entireties. These peptides and peptide analogues can be composed of L-amino acid or D-amino acids or mixture of Land D-amino acids. They may also include one or more non-peptide or amide linkages, such as one or more well-known peptide/amide isosteres. Such "peptide and/or peptide mimetic" apolipoproteins can be synthesized or manufactured using any technique for peptide synthesis known in the art, including, e.g., the techniques described in U.S. Pat. Nos. 6,004,925; 6,037,323 and 6,046,166.

The reconstituted HDL of the invention may include a single type of apolipoprotein, which may be derived from the same or different species. Although not required, the negatively charged lipoprotein complexes will preferably comprise apolipoproteins that are derived from, or correspond in amino acid sequence to, the animal species being treated, in order to avoid inducing an immune response to the therapy. The use of peptide mimetic apolipoproteins may also reduce or avoid an immune response.

The apolipoprotein may be any apolipoprotein which is a functional, biologically active component of naturally-occurring HDL or of a reconstituted high density lipoprotein (rHDL). Typically, the apolipoprotein is either a plasma-derived or recombinant apolipoprotein such as Apo-AI, Apo-AII or Apo-AV. Also contemplated are biologically-active fragments of the apolipoprotein. Fragments may be naturally occurring, chemical synthetic or recombinant. By way of example only, a biologically-active fragment of Apo-AI preferably has at least 50%, 60%, 70%, 80%, 90% or 95-100% or even greater than 100% of the LCAT stimulatory activity of Apo-AI when formulated in a rHDL composition.

In one embodiment of the invention, the apolipoprotein is at a concentration of about 5-100 g/L, preferably 10-50g/L or more preferably 25-45g/L. This includes 5, 10, 15, 20, 25, 30, 35, 40, 45, 50, 55, 60, 65, 70, 75, 80, 85, 90, 95 and 100 g/L and any ranges between these amounts. In other embodiments, the apolipoprotein may be at a concentration from about 5 to 20 g/L, e.g. about 8 to 12 g/L.

In one embodiment of the invention, the negatively charged phospholipid (PL) includes but is not limited to: phosphatidyl serine (PS), phosphatidyl inositol (PI), phosphatidyl glycerol (PG), preferably PS.

In one embodiment, the rHDL composition of the invention further comprises at least one zwitterionic phospholipid, preferably phosphatidyl choline (PC) or lecithin.

In another embodiment of the invention said zwitterionic phospholipid is phosphatidyl ethanolamine (PE).

PC, PS, PI and PG may represent natural or synthetic mixture or combination of individual molecular species of different fatty acid composition, such as, for example, egg PC, egg PG, soy bean PC, hydrogenated soy bean PC, soy bean PG, brain PS. Synthetic derivatives include dipalmitoylphosphatidylcholine (DPPC), didecanoylphosphatidylcholine (DDPC), dierucoylphosphatidylcholine (DEPC), dimyristoylphosphatidylcholine (DMPC), distearoylphosphatidylcholine (DSPC), dilaurylphosphatidylcholine (DLPC), palmitoyloleoylphosphatidylcholine (POPC), palmitoylmyristoylphosphatidylcholine (PMPC), palmitoylstearoylphosphatidylcholine (PSPC), dioleoylphosphatidylcholine (DOPC), dilauroylphosphatidylglycerol (DLPG), distearoylphosphatidylglycerol (DSPG), dimyristoylphosphatidylglycerol (DMPG), dipalmitoylphosphatidylglycerol (DPPG), distearoylphosphatidylglycerol (DSPG), dioleoylphosphatidylglycerol (DOPG), palmitoyloleoylphosphatidylglycerol (POPG), dimyristoylphosphatidylserine (DMPS), dipalmitoylphosphatidylserine (DPPS), dioleoylphosphatidylserine (DOPS) or may include purified molecular species of a given fatty acid composition only.

In one embodiment of the invention, the reconstituted HDL comprises phosphatidyl serine (PS) and phosphatidyl choline (PC).

In another embodiment of the invention, the reconstituted HDL comprises PI and PC.

In another embodiment of the invention, the reconstituted HDL comprises PG and PC.

In another embodiment of the invention, the negatively charged phospholipid is not phosphatidic acid (PA).

In one embodiment of the invention, the rHDL composition of the invention comprises 1 to 5 molecules of apolipoprotein.

In another embodiment, the rHDL composition of the invention comprises 2 to 300 lipid molecules, 10 to 150 molecules, 20 to 100 molecules, 20 to 75 molecules and most preferably 20 to 50 lipid molecules.

In one embodiment, the molar ratio of Apo-AI: negatively charged-phospholipid in the rHDL composition of the invention is about 1:40 to 1:55.

In another embodiment where the rHDL composition of the invention comprises PC and PS, the ratio PC:PS is about 5:1 ; 4:1 ; 3:1 ; 2:1 ; 1:1, preferably 1:1.

In another embodiment where the rHDL composition of the invention is naturally occurring HDL enriched in negatively charged phospholipids such as PC and/or PS, the rHDL contains about 5% of negatively charged phospholipids relative to total phospholipid.

It is expected that the inclusion of negatively charged phospholipids in the reconstituted HDL of the invention will provide the particles with greater stability (in solution) and longer product shelf-life compared to conventional particles. In addition, the use of negatively charged phospholipids is expected to minimize particle aggregation (e.g., by charge repulsion), thereby effectively increasing the number of available particles present in a given dosage regime, and aid the targeting of the particle for recognition.

Some apolipoproteins exchange in vivo from one reconstituted HDL to another (this is true for apolipoprotein ApoA-I). During the course of such exchange, the apolipoprotein typically carries with it one or more phospholipid molecules. Owing to this property, it is expected that the reconstituted HDL described herein will "seed" negatively charged phospholipids to endogenous HDL, thereby transforming them into alpha particles that are more resistant to elimination by the kidneys. Thus, it is expected that administration of the reconstituted HDL and compositions described herein will increase serum levels of HDL, reduce inflammation and/or improve endogenous HDL half-life as well as endogenous HDL metabolism. It is expected that this will result in improvement of cholesterol metabolism, reverse cholesterol transport and decreasing of inflammation.

In addition to the negatively charged phospholipids(s), the lipid fraction may optionally include additional lipids. Virtually any type of lipids may be used, including, but not limited to, lysophospholipids, galactocerebroside, gangliosides, cerebrosides, glycerides, triglycerides, and cholesterol and its derivatives.

When included, such optional lipids will typically comprise less than about 50 wt% of the lipid fraction, although in some instances more optional lipids could be included. In one embodiment, the lipid fraction of the reconstituted HDL of the invention does not include optional lipids.

In one embodiment of the invention, the rHDL composition may further comprise neutral lipids, such as cholesteryl esters.

In one embodiment of the invention, the rHDL composition may comprise a neutral phospholipid such as sphingomyelin.

In another embodiment of the invention, the rHDL composition does not comprise neutral phospholipids such as sphingomyelin.

Protein and lipid concentration of reconstituted HDL particles solution can be measured by any method known in the art, including, but not limited to, protein and phospholipid assays as well as by chromatographic methods such as HPLC, gel filtration chromatography, GC coupled with various detectors including mass spectrometry, UV or diode-assay, fluorescent, elastic light scattering and others. The integrity of lipid and proteins can be also determined by the same chromatographic techniques as well as peptide mapping, SDS-page gel, N- and C-terminal sequencing for proteins and standard assays to determine lipid oxidation for lipids.

The homogeneity and/or stability of the reconstituted HDL particles or composition of the invention can be measured by any method known in the art, including, but not limited to, chromatographic methods such as gel filtration chromatography. For example, a single peak or a limited number of peaks can be associated with a stable complex. The stability of the particles can be determined by monitoring the appearance of new peaks over time. The appearance of new peaks is a sign of reorganization among the particles due to the instability of the particles.

The optimum ratio of phospholipids to apolipoprotein(s) in the reconstituted HDL particles can be determined using any number of functional assays known in the art, including, but not limited to, gel electrophoresis mobility assay, size exclusion chromatography, interaction with HDL receptors, recognition by ATP-binding cassette transporter (ABCA1), uptake by the liver, and pharmacokinetics/pharmacodynamics. For example, gel electrophoresis mobility assays can be used to determine the optimum ratio of phospholipids to apolipoproteins in the charged complexes.

As another example, size exclusion chromatography can be used to determine the size of the reconstituted HDL particles of the invention as compared to natural pre-beta-HDL particles. Natural pre-beta-HDL particles generally are not larger than 10-12 nm, and discoidal particles are usually around 7-10 nm.

As another example, HDL receptors can be used in a functional assay to identify which complex is the closest to natural pre-beta-HDL particles, or to identify which complex is the most effective in removing and/or mobilizing cholesterol or lipids from a cell. In one assay, the complexes can be tested for their ability to bind ABCA1 receptors. Such an assay can differentiate ABCA1 dependent on independent removal of cholesterol. Even though ApoA-I is considered the best ligands for such an assay, complexes such as small micellar or small discoidal particles are also potent ABCA1 ligands. ABCA1 binding assays that can be used are described in Brewer et al., 2004, Arterioscler. Thromb. Vase. Biol. 24:1755- 1760.

In one embodiment, the reconstituted HDL of the invention further comprises a detergent.

In one embodiment of the invention, the level of detergent is about 5-35% of that which displays liver toxicity. This range includes, for example, 5%, 10%, 15%, 20%, 25%, 30% and 35%. Preferably, the level of detergent is about 5-20% of that which displays liver toxicity. Advantageously, the level is about 5-10% of that which displays liver toxicity. Most preferably, these levels are expressed in terms of the minimum or threshold level of detergent that displays liver toxicity.

By way of example, a level of detergent which has been shown in work leading to the present invention to cause, result in or at least be associated with liver toxicity is 0.3g/g Apo-AI or 6g/L rHDL composition (at 20g/L Apo-AI). Accordingly, 5-10% of this level of detergent is 0.015-0.03 g/g Apo-AI or 0.5-0.9 g/L rHDL composition (at 30g/L Apo-AI).

The "level" of detergent may be an absolute amount of detergent, a concentration of detergent (e.g. mass per unit volume of rHDL composition) and/or a ratio of the amount or concentration of detergent relative to another amount or concentration of a component of the rHDL composition. By way of example only, the level of detergent may be expressed in terms of the total mass of apolipoprotein (e.g. Apo-AI) present in the rHDL composition.

While safety and avoidance of liver toxicity is one object of the invention, the invention also requires a level of detergent sufficient to maintain rHDL composition stability. As will be described in more detail in the Examples, a detergent concentration no less than about 0.45 g/L of rHDL composition with 30 g/L apolipoprotein is optimal in terms of both stability and non-toxicity. Stability may advantageously be measured by any means known in the art, although turbidity of the rHDL composition is a preferred measure.

The detergent may be any ionic (e.g cationic, anionic, Zwitterionic) detergent or nonionic detergent, inclusive of bile acids and salts thereof, suitable for use in rHDL compositions. Ionic detergents may include bile acids and salts thereof, polysorbates (e.g PS80), CHAPS, CHAPSO, cetyl trimethyl-ammonium bromide, lauroylsarcosine, r/-octyl phenyl propanesulfonic acid and 4'-amino-7-benzamido-taurocholic acid.

Bile acids are typically dihydroxylated or trihydroxylated steroids with 24 carbons, including cholic acid, deoxycholic acid chenodeoxycholic acid or ursodeoxycholic acid. Preferably, the detergent is a bile salt such as a cholate, deoxycholate, chenodeoxycholate or ursodeoxycholate salt. A more preferred detergent is sodium cholate.

Liver toxicity can be measured by determining alanine aminotransferase activity, aspartate aminotransferase activity, and/or bilirubin's levels in plasma.

In another embodiment of the invention, the reconstituted HDL of the invention may further comprise a stabilizer. In particular, the stabilizer maintains stability of the rHDL composition during lyophilisation. Preferably, the stabilizer is a carbohydrate such as a sugar or sugar alcohol. Examples of suitable sugar alcohols are mannitol and sorbitol. In one embodiment of the invention, the stabilizer comprised in the reconstituted HDL composition is a disaccharide sugar such as sucrose.

The concentration of sucrose is about 65-85 g/L (equivalent to about 6.5-8.5% w/v) of the reconstituted HDL of the invention. Preferably, the concentration of sucrose is about 75 g/L (equivalent to about 7.5% w/w). It is proposed that this relatively reduced sucrose may allow for a faster infusion rate of the reconstituted HDL of the invention. Other stabilizers may be or include amino acids (e.g. glycine, proline), antioxidants, emulsifiers, surfactants, chelating agents, gelatine, synthetic oils, polyols, alginate or any pharmaceutically acceptable carriers and/or excipients, although without limitation thereto. In this regard, reference is made by way of example to "Pharmaceutical Formulation Development of Peptides and Proteins", Frokjaer et al., Taylor &; Francis (2000), "Handbook of Pharmaceutical Excipients", 3rd edition, ibbe et al., Pharmaceutical Press (2000) and International Publication WO2009/025754.

In one preferred embodiment, the reconstituted HDL of the invention comprises:
(i) about 30g/L Apo-A1;
(ii) about 0.03g sodium cholate per gram Apo-A1;
(iii) about 34, 40 or 47g/L PL, comprising a neutral lipid and/or a zwitterionic PL such as PC and negatively charged PL, most preferably PS; and
(iv) about 75g/L sucrose;
wherein the molar ratio of Apo-A1:total phospholipid is about 1:40 or 1:55.

Another object of the invention is a method to produce the reconstituted HDL of the invention comprising Apolipoprotein or fragments or mixtures thereof and at least one negatively charged phospholipid or fragments or mixtures thereof.

In a preferred embodiment of the method, an initial of starting level of detergent is reduced or removed to a level which does not display liver toxicity upon administration of the reconstituted HDL of the invention to a human.

Reduction or removal of detergent may be performed by any means known in the art including filtration, hydrophobic adsorption or hydrophobic interaction chromatography, dialysis, ion-exchange adsorption and ion-exchange chromatography, for example.

In some embodiments, non-polar polystyrene resins may be suitable for reducing detergent levels. Such resins preferably are in the form of a cross-linked copolymer (e.g. a cross-linked styrene and divinylbenzene copolymer). Non-limiting examples include Amberlite XAD-2 and Bio Beads SM.

Filtration includes gel filtration, gel permeation, diafiltration and ultrafiltration, although without limitation thereto, as are well understood in the art. A non-limiting example of gel permeation may utilize porous, cross-linked dextran such as Sephadex resins.

In one embodiment particularly suitable for large scale manufacture, the detergent level is reduced by diafiltration.

In one embodiment of the invention, the method includes the step of combining the lipid and the apolipoprotein in the absence of organic solvent.

In one embodiment, the invention provides a method of producing a reconstituted HDL including the steps of:
a. adding negatively charged PL and/or a neutral lipid and/or a zwitterionic PL, without organic solvent and a cholate detergent to an Apo-Al solution;
b. reducing the level of cholate detergent in the solution produced at step (a) to about 0.03g/g Apo-Al ;
c. adding a stabilizer, preferably sucrose, to the solution at step (b). Preferably, at step (a), the negatively charged PL is added so that the Apo-A1:total PL ratio is about 1:40 or 1:55.

Preferably, the final concentration of sucrose at step (c) is about 75 g/L.

In one embodiment of the invention, the method further includes the step (d) of lyophilizing the reconstituted HDL produced at step (c).

It will be appreciated that in a particular embodiment the method of producing a reconstituted HDL is suitable for large scale, commercial manufacturing of a reconstituted HDL of a quality and safety suitable for administration to humans.

Another object of the invention is a medicament comprising at least one reconstituted HDL of the invention as described here above for use in the treatment of a disorder related to dysfunction of HDL metabolism, an inflammatory disorder or a CV disorder. Examples of disorders related to dysfunction of HDL metabolism include but are not limited to stroke, ischemic stroke, transient ischemic attack, myocardial infraction, angina pectoris, inflammatory disorder, CV-related diseases and/or metabolic-related diseases: type 2 diabetes, metabolic syndrome, atherosclerosis, premature atherosclerosis, hyperlipidemia, especially hypercholesterolemia, familial hypercholesterolemia, familial combined hyperlipidemia, coronary heart disease, coronary artery disease, acute coronary syndrome, vascular and perivascular diseases, renovascular insufficiency, critical limb ischemia, rest pain, gangrene, restenosis; dyslipidemic disorders; dyslipoproteinemia; high levels of low density lipoprotein cholesterol; high levels of very low density lipoprotein cholesterol; low levels of high density lipoproteins; high levels of lipoprotein Lp(a) cholesterol; high levels of apolipoprotein B; familial combined hyperlipidemia (FCH); lipoprotein lipase deficiencies, such as hypertriglyceridemia and hypoalphalipoproteinemia. Syndromes associated with atherosclerosis such as intermittent claudication, caused by arterial insufficiency, are also included.

Examples of inflammatory disorders include but are not limited to inflammatory myocardial infarction, chronic vascular inflammation, inflammatory angiogenesis, pulmonary arterial hypertension, acute coronary syndromes, atherosclerosis, carotid thrombosis, ischemia, vasculitis, lupus, arthritis, psoriasis.

Examples of CV disorders include but are not limited to: coronary artery disease (also known as coronary heart disease and ischemic heart disease) cardiomyopathy; hypertensive heart disease, heart failure, cardiac dysrhythmias, inflammatory heart disease, endocarditis, inflammatory cardiomegaly, myocarditis, valvular heart disease, cerebrovascular disease, stroke, ischemia, peripheral arterial disease, congenital heart disease (heart structure malformations existing at birth), rheumatic heart disease.

In one embodiment, the form of the medicament, the route of administration, the dosage and the regimen naturally depend upon the condition to be treated, the severity of the illness, the age, weight, and sex of the patient, etc.

Toxicity and therapeutic efficacy of the rHDL composition can be determined using standard pharmaceutical procedures in cell culture or experimental animals for determining the LD50 (the lethal dose to 50% of the population) and the ED50 (the therapeutically effective dose in 50% of the population). The dose ratio between toxic and therapeutic effects is the therapeutic index and it can be expressed as the ratio LD50/ED50. Charged lipoprotein complexes that exhibit large therapeutic indices are preferred. Non-limiting examples of parameters that can be followed include liver function transaminases (no more than 2X normal baseline levels). This is an indication that hepatic cholesterol metabolism is perturbed by exogenous rHDL. The effect on red blood cells could also be monitored, as mobilization of cholesterol from red blood cells causes them to become fragile, or affect their shape.

Another object of the invention is the rHDL composition for use in the treatment of a disorder related to dysfunction of HDL metabolism, an inflammatory disorder or a CV disorder, wherein the rHDL composition will be formulated for an administration to a subject in need thereof.

The rHDL composition of the present invention may be administered orally, parenterally, by inhalation spray, topically, rectally, nasally, buccally, vaginally or via an implanted reservoir. The term administration used herein includes subcutaneous, intravenous, intramuscular, intra-articular, intra-synovial, intrasternal, intrathecal, intrahepatic, intralesional and intracranial injection or infusion techniques.

Examples of compositions adapted to oral administration include, but are not limited to, solid forms, liquid forms and gels. Examples of solid forms adapted to oral administration include, but are not limited to, pill, tablet, capsule, soft gelatine capsule, hard gelatine capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

Sterile injectable forms of the compositions of this invention may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the composition of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of composition.

According to one embodiment of the invention, the composition, the pharmaceutical composition or the medicament of the invention is administered at a dose determined by the skilled artisan and personally adapted to each subject.

In one embodiment, the fixed dosage rHDL composition is at a dosage that is therapeutically effective upon administration to subjects of any body weight or of any body weight in a body weight range. Accordingly, the rHDL composition dosage is not calculated, determined or selected according to the particular body weight of the subject, such as would typically occur with "weight-adjusted dosing".

In another embodiment, the fixed dosage rHDL composition is determined as a dosage which when administered to subjects of any body weight or of any body weight in a body weight range, would display relatively reduced inter-subject variability in terms of exposure to the rHDL composition. Relatively reduced inter-subject variability is compared to that observed or associated with weight-adjusted dosing of a subject population.

Variability of exposure may be expressed or measured in terms of the variation in exposure of patients to rHDL following administration of the fixed dosage rHDL composition. Preferably, the variability is that which would occur when the fixed dosage rHDL composition is administered to subjects over a weight range compared to the variability that would occur for weight-adjusted dosages administered to subjects over the same weight range as the fixed dosage subjects. In some embodiments, exposure to apolipoprotein may be measured as average exposure (e.g. mean or median exposure), total exposure (e.g. amount integrated over time of exposure) or maximum exposure level (e.g. Cmax). Generally, the weight or weight range is 20, 30, 40, 50, 60, 70, 80, 90, 100, 110, 120, 130, 140, 150, 160, 170, 180, 190 or 200 kg, or any range between these values. Preferably, the weight or weight range is 20-200 kg, 20-60 kg, 40-160 kg, 50-80 kg, 60-140 kg, 70-80 kg, 80-120 kg, 100-180 kg or 120-200 kg.

In one embodiment, the variability is less than 100% or preferably 99%, 98%, 97%, 96% 95%, 94%, 93%, 92%, 91%, or less than 90%, 85% or 80% of the variability that occurs with weight-adjusted dosing. Variability may be calculated and expressed by any statistical representation known in the art, including as a coefficient of variation (e.g. %CV), standard deviation, standard error or the like, although without limitation thereto.

Notwithstanding administration of a fixed dosage rHDL composition to subjects of markedly different body weights, the exposure of the subjects to apolipoprotein is surprisingly uniform. Accordingly it is proposed that the therapeutic efficacy of the fixed dosage rHDL composition will not be substantially compromised or reduced compared to a weight-adjusted dosage.

By way of example only, there is no difference in total exposure to apolipoprotein upon administration of a fixed dosage rHDL composition to subjects in the 60-120 kg weight range. Furthermore, Cmax for apolipoprotein decreased by an average of 16% over the 60- 120 kg weight range.

In comparison, for weight-adjusted dosing regimens using the same rHDL composition, a doubling of body weight from 60 kg to 120 kg requires a doubling of the dosage of apolipoprotein and increased apoA-I exposures.

Fixed dosage rHDL compositions may be administered in multiple doses at any suitable frequency including daily, twice weekly, weekly, fortnightly or monthly.

Preferred fixed dosages include 0.1-15g, 0.5-12g, 1-10g, 2-9g, 3-8g, 4-7g or 5-6g of apolipoprotein. Particularly preferred fixed dosages include l-2g, 3-4g, 5-6g or 6-7g of apolipoprotein. Non-limiting examples of specific fixed dosages include 0.25g, 0.5g, lg, 1.7g, 2g, 3.4g, 4g, 5.1g, 6g, 6.8g and 8g of apolipoprotein.

Non-limiting, specific examples of fixed dosage administration regimens that may be employed include 0.25g, 0.5g, lg, 1.7g, 2g, 3.4g, 4g, 5.1g, 6g, 6.8g or 8g weekly by intravenous infusion over 90 min, 0.25g, 0.5g, lg, 1.7g, 2g, 3.4g, 4g, 5.1g, 6g, 6.8g or 8g apolipoprotein weekly by intravenous infusion over 120 min or 0.25g, 0.5g, 1g, 1.7g, 2g, 3.4g, 4g, 5.1g, 6g, 6.8g or 8g apolipoprotein twice weekly by intravenous infusion over 90 min or over 120 min.

In one embodiment, the rHDL composition of the invention may be administered at a dosage in the range of 1-120 mg/kg body weight. Preferably, the dosage is in the range of 5-80 mg/kg inclusive of 10 mg/kg, 20 mg/kg, 30 mg/kg, 40 mg/kg, 50 mg/kg, 60 mg/kg, and 70 mg/kg.

In one embodiment of the invention, the subject is affected by a disorder related to dysfunction of HDL metabolism.

In one embodiment of the invention, the subject is affected by an inflammatory disorder.

In one embodiment of the invention, the subject is affected by a CV disorder.

In one embodiment of the invention, the subject has been diagnosed with a disorder related to dysfunction of HDL metabolism.

In one embodiment of the invention, the subject has been diagnosed with an inflammatory disorder.

In one embodiment of the invention, the subject has been diagnosed with a CV disorder.

In one embodiment of the invention, the subject is at risk of developing a disorder related to dysfunction of HDL metabolism.

In one embodiment of the invention, the subject is at risk of developing an inflammatory disorder.

In one embodiment of the invention, the subject is at risk of developing a CV disorder.

In one embodiment of the invention, the subject is a mammal and preferably a human.

In one embodiment of the invention, the subject is a female. In another embodiment of the invention, the subject is a male.

One object of the invention is a method for enriching negatively charged phospholipid in a small dense HDL particle in a subject in need thereof, comprising administering to the subject a therapeutically effective amount of the composition of the invention.

One object of the invention is a method for clearing arterial wall cholesterol in a subject in need thereof, comprising administering to the subject a therapeutically effective amount of the composition of the invention.

One object of the invention is a method for reducing oxidative stress in a subject in need thereof, comprising administering to the subject a therapeutically effective amount of the composition of the invention.

One object of the invention is a method for reducing thrombosis in a subject in need thereof, comprising administering to the subject a therapeutically effective amount of the composition of the invention, wherein said reduction of thrombosis is obtained by reduction of platelet aggregation.

One object of the invention is a method for reducing inflammation in a subject in need thereof, comprising administering to the subject a therapeutically effective amount of the composition of the invention. As for example, the level of inflammatory cytokines such as IL-6 and TNF-α may be reduced when using the composition of the invention.

One object of the invention is a method for reducing CV inflammation in a subject in need thereof, comprising administering to the subject a therapeutically effective amount of the composition of the invention. As for example, the level of inflammatory cytokines such as IL-6 and TNF-alpha may be reduced when using the composition of the invention.

One object of the invention is a method for reducing cellular apoptosis in a subject in need thereof, comprising administering to the subject a therapeutically effective amount of the composition of the invention.

One object of the invention is a method for improving glucose metabolism in a subject in need thereof, comprising administering to the subject a therapeutically effective amount of the composition of the invention. As for example, apoptosis may be reduced in endothelial cells and macrophages.

Another object of the invention is a method for treating a disorder related to the dysfunction of HDL metabolism in a subject in need thereof, comprising administering to the subject a therapeutically effective amount of the composition of the invention.

Another object of the invention is a method for treating an inflammatory disorder in a subject in need thereof, comprising administering to the subject a therapeutically effective amount of the composition of the invention.

The invention also provides a fixed dosage, rHDL kit comprising one or more unit doses of the fixed dosage rHDL composition disclosed herein and one or more other kit components.

In one embodiment of the invention, the kit is for prophylactically or therapeutically treating a disorder in a subject, as hereinbefore described.

Non-limiting examples of one or more other kit components include instructions for use; vials, containers or other storage vessels containing each of the unit doses; delivery devices such as needles, catheters, syringes, tubing and the like; and/or packaging suitable for safely and conveniently storing and/or transporting the kit. Preferably the instructions for use are a label or package insert, wherein the label or package insert indicates that the rHDL composition of the invention may be used to treat a disorder by administering a fixed dose amount to a subject in need thereof.

A "package insert" refers to instructions included in commercial packages of the rHDL compositions, that contains information about the indications, usage, dosage, administration, contraindications and/or warnings concerning the use of such rHDL compositions.

For the purposes herein, a "vial" refers to a container which holds the rHDL composition of the invention. The vial may be sealed by a stopper pierceable by a syringe. Generally, the vial is formed from a glass material. Accordingly, a vial preferably comprises the lyophilized rHDL composition with protein content of 0.25g, 0.5g, 1g, 2g, 2.5g, 3g, 3.5g, 4g, 4.5g, 5g, 5.5g, 6g, 6.5g, 7g, 8g or 10 g per vial. More preferably the apolipoprotein content is either 0.5g, 1g, 2g, 4g, 6g, 8g or 10 g per vial.

The rHDL composition in the vial can be in various states including liquid, lyophilized, frozen etc. The fixed dosage rHDL composition is preferably stable as a liquid. Stability may be measured by any means known in the art, although turbidity is a preferred measure. Turbidity level of below about 10, 15, 20, or 30 Nephelometric Turbidity Unit (NTU) can generally be considered a stable fixed dosage rHDL composition. Turbidity measurements can be taken by incubating the fixed dosage rHDL compositions over time periods such as 0 hr, 2 hr, 4hr, 6 hr, 12 hr, 18 hr, 24 hr, 36 hr, 72 hr, 7 days and 14 days at storage temperatures such as room temperature or 37°C. Preferably the fixed dosage rHDL composition is considered to be stable as a liquid when it is stored for 14 days at room temperature and exhibits a turbidity of less than about 15 NTU.

The kit may facilitate administration of the fixed dosage rHDL composition by a person skilled in the art or self-administration by a subject or caregiver.

Accordingly, the fixed dosage rHDL composition may generally apply to any such composition that would usually be administered by "weight-adjusted" dosing.

### BRIEF DESCRIPTION OF THE DRAWINGS

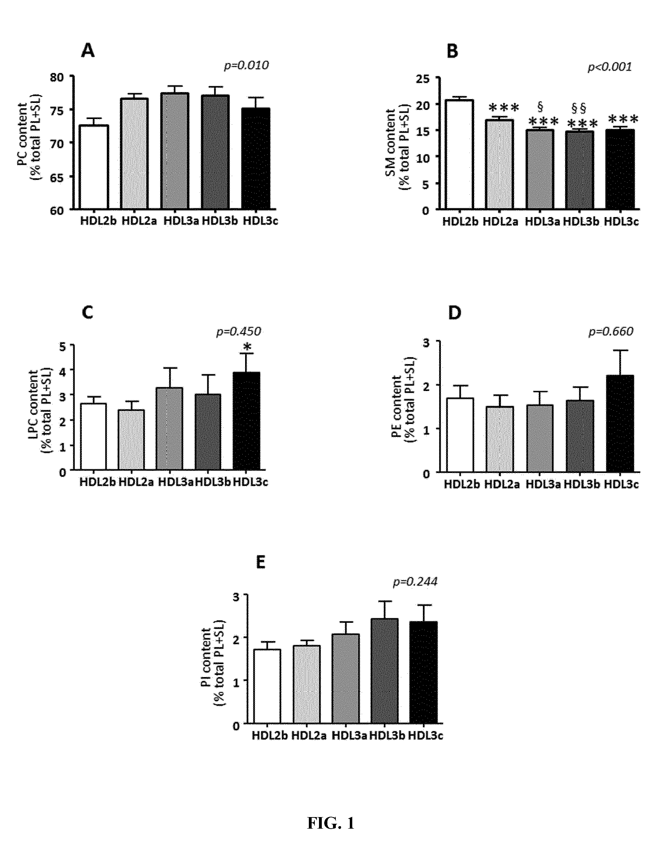

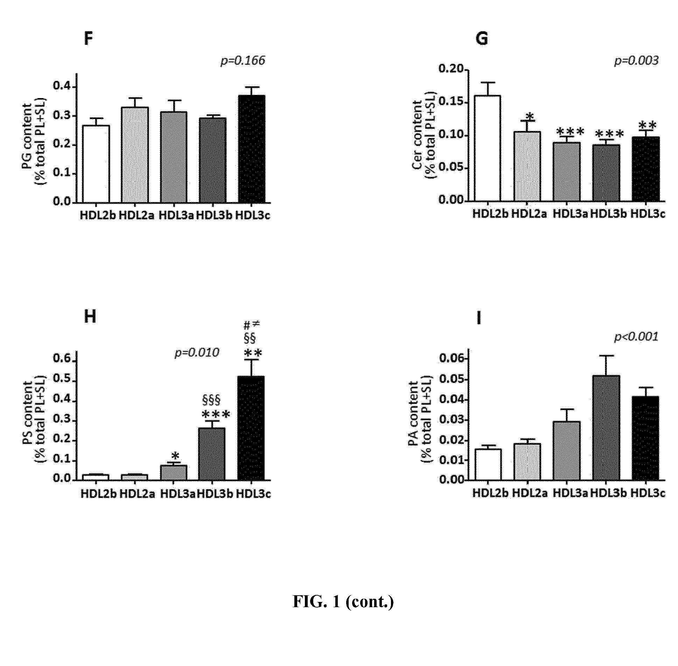

**Figure 1** represents histograms showing phosphosphingolipidome of HDL subpopulations in healthy normolipidemic subjects. HDL contents of PC (A), SM (B), LPC (C), PE (D), PI (E), PG (F), Cer (G), PS (H) and PA (I), expressed as wt % of total phosphosphingolipidome in each HDL subclass, are shown in the order of decreasing abundance; *p<0.05, **p<0.01, ***p<0.001 vs. HDL2b; §p<0.05, §§p<0.01, §§§p<0.001 vs. HDL2a; #p<0.05 vs. HDL3a; ≠p<0.05 vs. HDL3b. In the upper right corner, p-values for the trend between HDL subpopulations are shown.

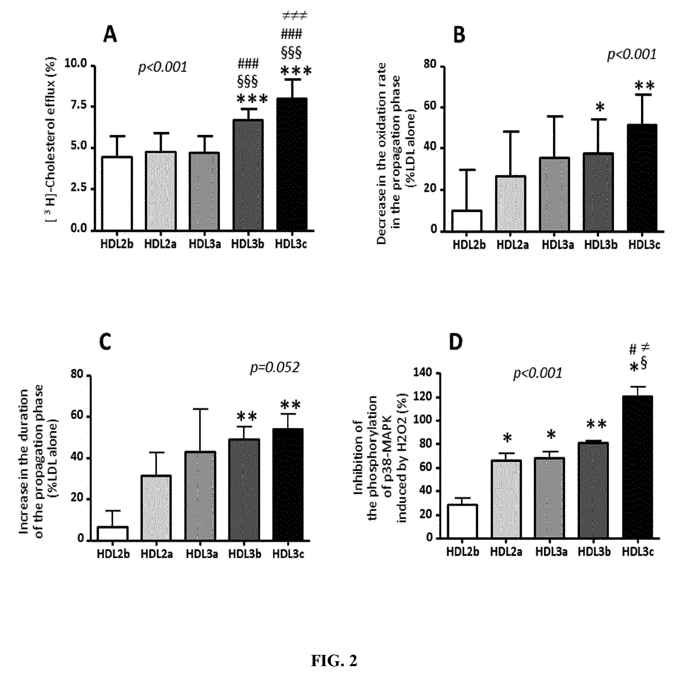

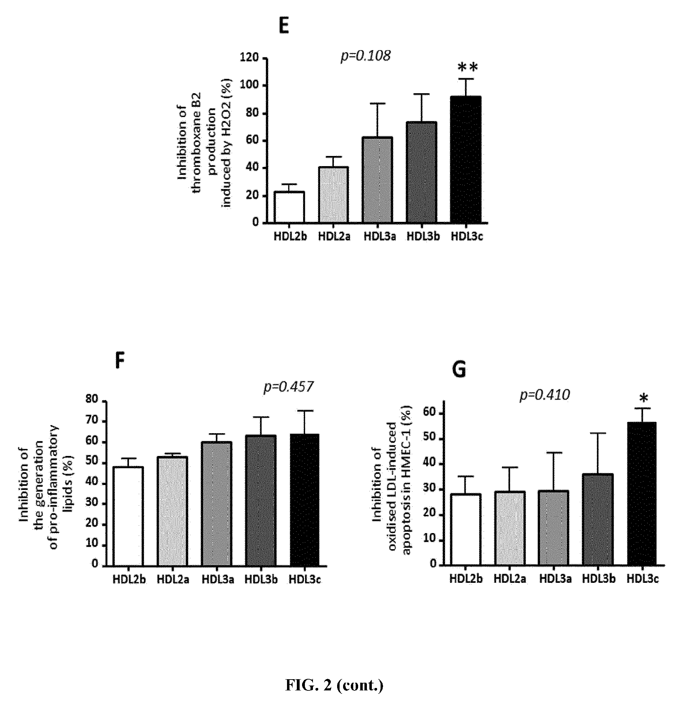

**Figure 2** represents histograms showing biological activities of HDL subpopulations in healthy normolipidemic subjects. Cholesterol efflux capacity in THP-1 cells expressed as % [3H]-cholesterol efflux to HDL compared on the basis of unit PL mass content (A). Anti-oxidative activity of HDL compared on the basis of total mass content towards LDL oxidation, expressed as a decrease in the LDL oxidation rate in the propagation phase (B) and an increase in the duration of this phase (C). Anti-thrombotic activity of HDL compared on the basis of total protein content, expressed as an inhibition of H2O2-induced p38-MAPK phosphorylation (D) and thromboxane B2 production (E) in human platelets. Cell-free anti-inflammatory activity of HDL compared on the basis of total mass content, expressed as an attenuation of the generation of pro-inflammatory oxidized PLs in LDL (F). Anti-apoptotic activity of HDL compared on the basis of total protein content, expressed as a protection from the inhibition of mitochondrial function in HMEC-1 induced by oxidized LDL (G). *p<0.05, **p<0.01, ***p<0.001 vs. HDL2b; §p<0.05, §§§p<0.001 vs. HDL2a; ###p<0.001, #p<0.05 vs. HDL3a; ≠≠≠p<0.001, ≠p<0.05 vs. HDL3b. In the upper part, p-values for the trend between HDL subpopulations are shown.

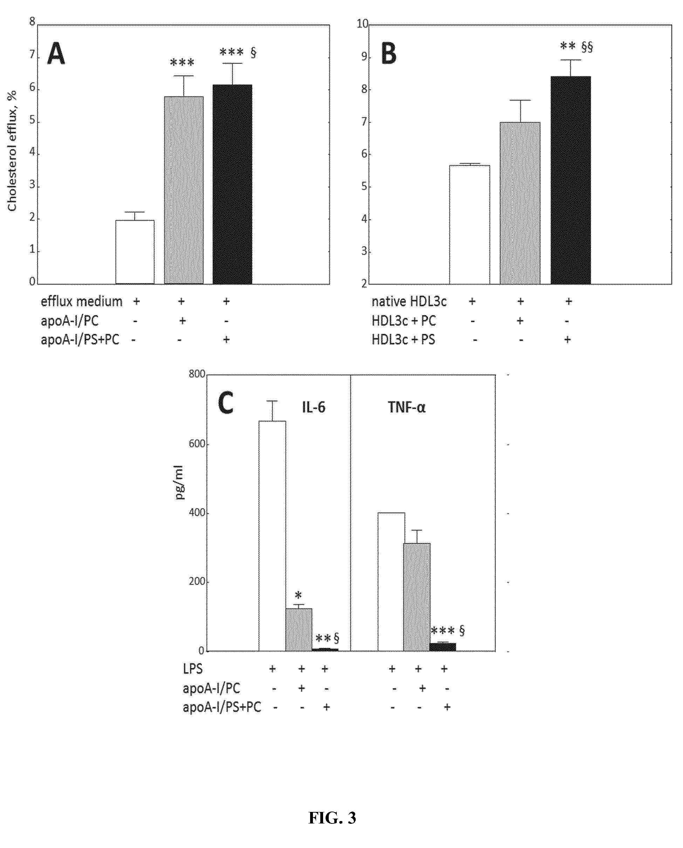

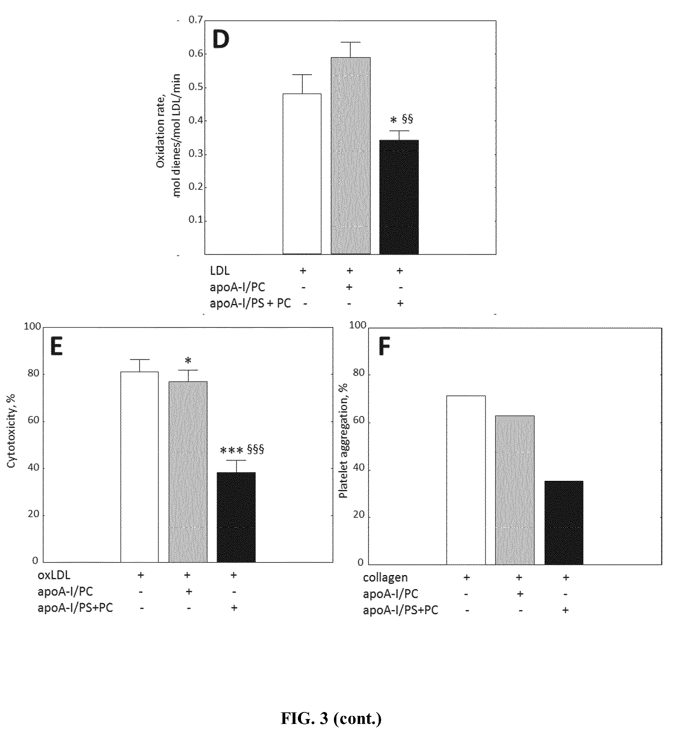

**Figure 3** represents histograms showing effects of rHDL containing ApoA-I and PC or Apo A-I, PC and PS on cholesterol efflux (A), inflammation (C), oxidative stress (D), cytotoxicity (E) and platelet aggregation (F). Cholesterol efflux capacity of human small, dense HDL3c in vitro enriched in PC or PS is also shown (B). Cholesterol efflux capacity was assessed in THP-1 cells and expressed as % [3H]-cholesterol efflux to HDL compared on the basis of unit PL mass content (A, B). Anti-inflammatory activity of HDL was assessed on the basis of total mass content and expressed as an attenuation of the production of IL-6 and TNF-alpha by macrophages activated by LPS (C). Anti-oxidative activity of HDL was assessed on the basis of total mass content towards LDL oxidation and expressed as a decrease in the LDL oxidation rate in the propagation phase (D). Anti-apoptotic activity of HDL was assessed on the basis of total protein content and expressed as a protection from the inhibition of mitochondrial function in HMEC-1 induced by oxidized LDL (E). Anti-thrombotic activity of HDL was assessed on the basis of total protein content and expressed as an inhibition of collagen-induced aggregation of human platelets (F). *p<0.05, **p<0.01, ***p<0.001 vs. control incubations; § p<0.05, §§ p<0.01, §§§ p<0.001 vs. rHDL containing PC alone (A, C-F) or PC-enriched HDL3c (B).

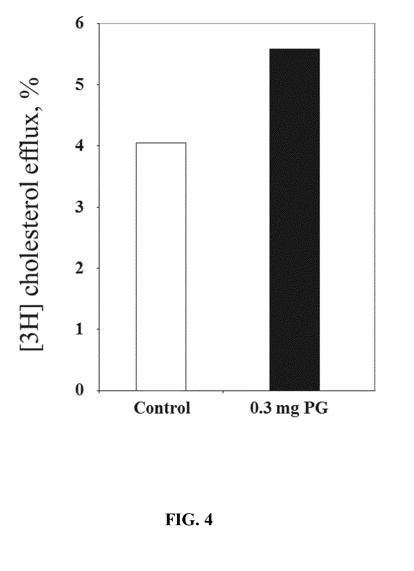

**Figure 4** represents a histogram showing effects of the enrichment in PG on cholesterol efflux capacity of native HDL. A representative experiment is shown out of two independent experiments.

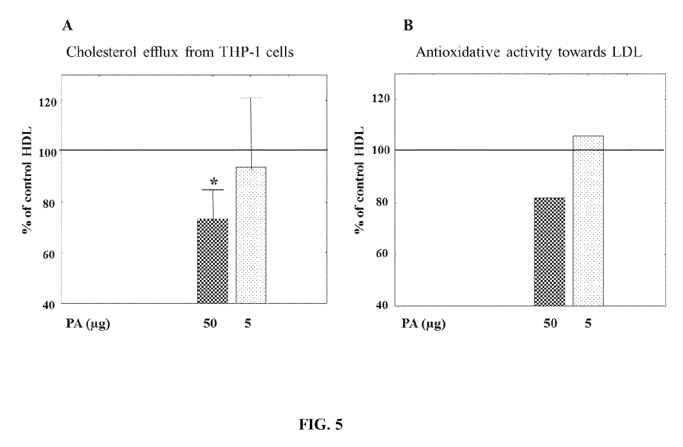

**Figure 5** represents histograms showing effects of the enrichment in PA on cholesterol efflux capacity (A) and anti-oxidative activity (B) of native HDL. A representative experiment out is shown of four independent experiments.

### EXAMPLES

The present invention is further illustrated by the following examples.

### Materials and Methods

### Subjects and blood samples

Fourteen normolipidemic healthy non-obese male volunteers were recruited at the La Pitié-Salpetrière University Hospital (Paris, France). All subjects were males between 32 and 67 years of age, non-smokers, and either abstainers or moderate alcohol consumers (<25 g/d). None of the subjects presented renal, hepatic, gastrointestinal, pulmonary, endocrine, or oncological disease or were receiving anti-oxidative vitamin supplementation or drugs known to affect lipoprotein metabolism for at least 6 weeks before the study. All subjects gave written informed consent. Venous blood was collected by venipuncture from the antecubital vein into sterile evacuated tubes (Vacutainer) in the presence or absence of ethylenediaminetetraacetic acid (K₃EDTA; final concentration, 1.8 mg/ml) after an overnight fast. After blood collection, EDTA plasma and serum were immediately separated by centrifugation at 4°C; sucrose (final concentration, 0.6%) was added as a cryoprotectant for lipoproteins and samples were aliquoted and stored at -80°C under nitrogen. Each aliquot was thawed only once directly before analyses.

### Clinical and biological parameters

Plasma levels of total cholesterol (TC), triglyceride (TG) and HDL-cholesterol (HDL-C) were measured using commercially available enzymatic kits. LDL-C was calculated using the Friedewald formula. Plasma apoA-I and apoB were quantitated by immunoturbidimetry. Systemic inflammation was assessed as the plasma level of high-sensitive C-reactive protein (hsCRP) measured by immunoassay.

### Fractionation of lipoproteins

Plasma lipoproteins were isolated from serum and plasma by single step, isopycnic non-denaturing density gradient ultracentrifugation in a Beckman SW41 Ti rotor at 40,000 rpm for 44 hours in a Beckman XL70 ultracentrifuge at 15°C by a slight modification of the method of Chapman et al. as previously described. After centrifugation, each gradient was fractionated in predefined volumes from the meniscus downwards with an Eppendorf precision pipette into 11 fractions corresponding to VLDL+IDL (d<1.019 g/mL), LDL (5 subfractions, LDL1, d 1.019-1.023 g/mL; LDL2, d 1.023-1.029 g/mL ; LDL3, d 1.029-1.039 g/mL; LDL4, d 1.039-1.050 g/mL; and LDL5, d 1.050-1.063 g/mL) and HDL. Five major HDL subclasses were isolated, i.e., large, light HDL2b (d 1.063-1.087 g/ml) and HDL2a (d 1.088-1.110 g/ml), and small, dense HDL3a (d 1.110-1.129 g/ml), HDL3b (d 1.129-1.154 g/ml) and HDL3c (d 1.154-1.170 g/ml). The validity and reproducibility of this density gradient procedure, which facilitates preparative fractionation of HDL particle subspecies in a non-denaturated, native state, have been extensively documented. All HDL subclasses isolated by this procedure are essentially albumin-free (<1% of total protein, i.e., <0.05 mg/dl). Lipoproteins were extensively dialyzed against phosphate-buffered saline (PBS; pH 7.4) at 4°C in the dark, stored at 4°C and used within 8 days.

### Preparation of reconstituted HDL (rHDL)

ApoA-I/PC and ApoA-I/PC/PS rHDL were prepared by the cholate dialysis method (Matz CE, Jonas A. Micellar complexes of human apolipoprotein A-I with phosphatidylcholines and cholesterol prepared from cholate-lipid dispersions. J Biol Chem. 1982; 257: 4535-40) as described previously (Rye KA, Hime NJ, Barter PJ. Evidence that cholesteryl ester transfer protein-mediated reductions in reconstituted high density lipoprotein size involve particle fusion. J Biol Chem. 1997; 272: 3953-60). Before use, rHDL were dialyzed against 0.01 mol/l Tris-buffered saline (pH 7.4) containing 0.15 mol/l NaCl, 0.005% EDTA-Na2 and 0.006% (w/v) NaN3 to remove cholate.

### Chemical analysis of lipoproteins

Total protein, TC, free cholesterol (FC), PL and TG contents of isolated lipoprotein subfractions were determined using commercially available assays. Cholesteryl ester (CE) was calculated by multiplying the difference between total and free cholesterol concentrations by 1.67. Total lipoprotein mass was calculated as the sum of total protein, CE, FC, PL and TG. ApoA-I and apoA-II content in HDL was quantitated using commercially available kits (Diasys, France).

### Phosphosphingolipidome analysis by mass spectrometry

Lipid standards. 1,2-dipalmitoyl-sn-glycero-3-phosphocholine-N,N,N-trimethyl-d9 (PC d9 16:0/16:0), 1-lauroyl-2-tridecanoyl-sn-glycero-3-phospho-(1'-myo-inositol) (PI 12:0/13:0), 1- dodecanoyl-2-tridecanoyl-sn-glycero-3-phosphoethanolamine (PE 12:0/13:0), 1-dodecanoyl-2-tridecanoyl-sn-glycero-3-phospho-(1'-rac-glycerol) (PG 12:0/13:0), 1-dodecanoyl-2-tridecanoyl-sn-glycero-3-phosphate (PA 12:0/13:0), 1-dodecanoyl-2-tridecanoyl-sn-glycero-3-phospho-L-serine (PS 12:0/13:0), 1-pentadecanoyl-sn-glycero-3-phosphocholine (LPC 15:0/0:0), and N-heptadecanoyl-D-erythro-sphingosine (Cer d18 :1/17:0) were used as internal standards. 1-Palmitoyl-2-hydroxy-*sn*-glycero-3-phosphocholine (LPC 16:0), 1-stearoyl-2-hydroxy-sn-glycero-3-phosphocholine (LPC 18:0), 1,2-dimyristoyl-sn-glycero-3-phosphocholine (PC 14:0/14:0), 1-myristoyl-2-palmitoyl-*sn*-glycero-3-phosphocholine (PC 14:0/16:0), 1,2-dipalmitoyl-*sn*-glycero-3-phosphocholine (PC 16:0/16:0), 1-palmitoyl-2-stearoyl-sn-glycero-3-phosphocholine (PC 16:0/18:0), 1-palmitoyl-2-oleoyl-*sn*-glycero-3-phosphocholine (PC 16:0/18:1), 1-palmitoyl-2-linoleoyl-*sn*-glycero-3-phosphocholine (PC 16:0/18:2), 1,2-distearoyl-*sn*-glycero-3-phosphocholine(PC 18:0/18:0), 1-stearoyl-2-oleoyl*sn*-glycero-3-phosphocholine (PC 18:0/18:1), 1-stearoyl-2-linoleoyl-*sn*-glycero-3-phosphocholine (PC 18:0/18:2), 1-stearoyl-2-arachidonoyl-sn-glycero-3-phosphocholine (PC 18:0/20:4), 1-palmitoyl-2-docosahexaenoyl-*sn*-glycero-3-phosphocholine (PC 16:0/22:6), 1-stearoyl-2-docosahexaenoyl-*sn*-glycero-3-phosphocholine (PC 18:0/22:6), 1-Stearoyl-2-Hydroxy-sn-Glycero-3-Phosphoethanolamine (LPE 18:0), 1,2-distearoyl-sn-glycero-3-phosphoethanolamine (PE 18:0/18:0), 1-heptadecanoyl-2-(9Z-tetradecenoyl)-sn-glycero-3-phospho-(1'-myo-inositol) (PI 17:0/14:1), N-stearoyl-D-*erythro*-sphingosine (Cer d18:1/18:0),1,2-distearoyl-sn-glycero-3-phosphate (PA 18:0/18:0), 1,2-distearoyl-**sn**-glycero-3-phospho (1'-rac-glycerol) (PG 18:0/18:0) and 1-palmitoyl-2-linoleoyl-**sn**-glycero-3-phospho-L-serine (PS 16:0/18:2) were purchased from Avanti Polar Lipids (Alabaster, AL, USA). LC/MS grade solvents were used without further purification and obtained from Sigma-Aldrich (St Louis,MO, USA) or VWR (West Chester, PA, USA).

Extraction. HDL subpopulations were extracted according to the following procedure. Briefly, 30µg of total phospholipid mass determined using a commercially available assay were added to 4ml of cold CHCl3/acidified CH3OH (5:2 v/v) containing 4µg of PC d9 32:0, 100ng of PI 25:0, 80ng of PE 25:0, 80ng of PA 25:0, 40ng of PS 25:0, 20ng of PG 25:0 and 20ng of Cer 17:0. A blank (PBS) and a control (HDL2 obtained from a reference normolipidemic plasma) sample was extracted in parallel with each batch to ensure for quality control; each samples were corrected for blank readings. K4EDTA (200mM) solution was added (1:5 v/v) and the mixture was vortexed for 1min and centrifuged at 3600 g for 10min at 4°C. The organic phase was transferred into 5ml chromacol glass tubes and dried under nitrogen. Lipids were reconstituted into 150µl isopropanol/hexane/water (10:5:2 v/v), transferred into LC/MS amber vials with inserts, dried under nitrogen and resuspended in 40µl of isopropanol/hexane/water (10:5:2 v/v). Molecular lipid species were analyzed and quantitated by LC/MS/MS.

LC/MS analysis. Seven principal PL subclasses (phosphatidylcholine (PC), lysophosphatidylcholine (LPC), phosphatidylethanolamine (PE), phosphatidylinositol (PI), phosphatidylglycerol (PG), phosphatidylserine (PS) and phosphatidic acid (PA)) and two principal sphingolipid (SL) subclasses (sphingomyelin (SM) and ceramide (Cer)), which together comprise >160 individual molecular lipid species and account for >95% of total plasma PL and SM, were assayed by LC/MS/MS. The lipid subclasses were divided into major (those whose content was >1% of total PL+SL, i.e. PC, SM, LPC, PE and PI) and minor (those whose content was <1% of total PL+SL, i.e. PG, Cer, PS and PA).

Lipids were quantified by LC-ESI/MS/MS using a QTrap 4000 mass spectrometer (AB Sciex, Framingham, MA, USA) equipped with a turbo spray ion source (300°C) combined with an LC20AD HPLC system, a SIL-20AC autosampler (Shimadzu, Kyoto, Japan) and the Analyst 1.5 data acquisition system (AB Sciex, Framingham, MA, USA). Quantification of PLs and SLs was performed in positive-ion mode, except for PI species that were detected in negative-ion mode. Sample (4µl) was injected onto a Symmetry Shield RP8 3.5µm 2.1x50mm reverse phase column (Waters Corporation, Milford,MA, USA) using a gradient from 85:15 to 91:9 (v/v) methanol/water containing 5mM ammonium formate and 0.1% formic acid at a flow rate of 0.1ml/min for 30 mins. Lipid species were detected using multiple reaction monitoring reflecting the headgroup fragmentation of each lipid class. PC, LPC and SM species were detected as product ions of m/z 184, PE, PS, PG and PA as neutral losses of respectively m/z 141, 185, 189 and 115, and PI molecular species as product ions of m/z -241. Air was used as nebulizing gas and N2 as collision gas. PE, PS, PG, PI, PA and ceramide species were monitored for 18ms; PC, LPC and SM species were monitored for 30ms at a unit resolution (0.7 atomic mass unit at half peak height).

Quantification. Lipids were quantified using calibration curves specific for the nine individual lipid classes with up to 12 component fatty acid moieties. Twenty-three calibration curves were generated in non-diluted and 10-fold diluted matrices to correct for matrix-induced ion suppression effects. More abundant lipid species which displayed a non-linear response in non-diluted extracts were quantified from a 10- or 100-fold diluted sample. An in-house developed Excel Macro script (Microsoft Office 2010, Redmond, WA, USA) was used to compile data from the three successive injections. Coefficients of variation (CVs) for analyzed lipids evaluated in one HDL sample isolated from a normolipidemic plasma pool were <10% for analyzed all lipid subclasses. The sample was analyzed 10 times in a row for intra-assay precision.

### Cellular cholesterol efflux capacity of HDL

The cholesterol efflux capacity of HDL subpopulations were characterized in a human THP-1 monocytic cell system (ATCC, Manassas, VA, USA) as previously described (Larrede S et al., 2009 Arterioscler Thromb Vasc Biol 29:1930-1936). In brief, THP-1 monocytes were cultured on 24-well tissue culture plates, grown in RPMI 1640 media with 10% FBS and differentiated into macrophage-like cells with 50 ng/ml phorbol 12-myristate 13-acetate (PMA) for 48 hours and 37°C. The cells were washed and loaded for 24 h with [3H]cholesterol-labelled acetylated LDL (acLDL, 1 µCi/mL) in serumfree RPMI 1640 culture medium supplemented with 50 mM glucose, 2 mM glutamine, 0.2% BSA, 100 µg/ml penicillin and 100 µg/ml streptomycin (further abbreviated as RGGB) to allow equilibration of cellular cholesterol pools. The labelling medium was removed and human macrophages were then equilibrated in RGGB for an additional 16-24 h period. Cellular cholesterol efflux to 15 µg/ml HDL-PL was assayed in serum-free medium for a 4-hour chase period. Finally culture media were harvested and cleared of cellular debris by brief centrifugation. Cell radioactivity was determined by extraction in hexane-isopropanol (3:2), evaporation of the solvent under nitrogen and liquid scintillation counting (Wallac Trilux 1450 Microbeta, Perkin Elmer, USA). The percentage of cholesterol efflux was calculated as (medium cpm) / (medium cpm + cell cpm) x 100%. Specific cholesterol efflux was determined by subtracting non-specific cholesterol efflux occurring in the absence of cholesterol acceptors.

### Anti-oxidative activity of HDL

Anti-oxidative activity of serum-derived HDL subpopulations (final concentration of each, 10 mg total mass/dl) was assessed at a physiological HDL to LDL ratio of 2-6 mol/mol towards reference LDL (d 1.019-1.063 g/ml; final concentration, 10 mg TC/dl) isolated from one healthy normolipidemic control subject. 3, 4, 11 HDL subfractions were added to LDL directly before oxidation. Lipoprotein oxidation was induced by an azo-initiator 2,2'-azo-bis-(2-amidinopropane) hydrochloride (AAPH; final concentration 1 mmol/l) (Kontush A, 2003 Arterioscler Thromb Vasc Biol 23:1881-1888) as a model of mild oxidation induced by free radicals in the arterial intima (Stocker R, 1994 Curr Opin Lipidol. Dec;5(6):422-33). Serum was used as a source of HDL for this assay to ensure intact paraoxonase activity, which is inhibited by EDTA. Thereby this assay employs mild oxidative conditions and integrates the antioxidative activities of several HDL components, i.e. apoA-I, anti-oxidative enzymes and lipophilic low-molecular-weight antioxidants. Accumulation of conjugated dienes was measured as the increment in absorbance at 234 nm. Absorbance kinetics were corrected for the absorbance of AAPH itself run in parallel as a blank. The kinetics of diene accumulation revealed two characteristic phases, the lag and propagation phases. For each curve, the duration of each phase, average oxidation rates within each phase and amount of dienes formed at the end of the propagation phase (maximal amount of dienes) were calculated.

### Anti-thrombotic activity

Anti-thrombotic activity of HDL particles was evaluated as their capacity to inhibit activation of human platelets. Platelets were isolated from blood collected at the blood bank from healthy volunteers who had not ingested any aspirin or other nonsteroidal anti-inflammatory drugs in the previous 10 days. 12 Platelets were preincubated with HDL (80 µg protein/ml) for 2h at 37°C and activated by H2O2 (200 µM) for 10 min at 37°C. Following platelet lysis, proteins (30 µg) were denatured, electrophoresed in 12% Bis-Tris gels (Bio-Rad Laboratories, Marnes-la-Coquette, France) and transferred to nitrocellulose membranes. The membranes were incubated with an anti-phospho p38 MAPK antibody or an anti-p38 MAPK polyclonal antibody (Cell Signaling Technologies, Beverly, MA) diluted 1:5000, washed and incubated with 1:10000 goat anti-rabbit horseradish peroxidase conjugate. p38-MAPK was visualized by enhanced chemiluminescence, and bands were quantified by densitometry (Amersham Biosciences, Buckinghamshire, UK). Platelet thromboxane B2 was quantified by competitive enzyme immunoassay (Amersham Biosciences, Buckinghamshire, UK).

### Anti-inflammatory activity

Anti-inflammatory activity of HDL was assessed as the capacity to prevent the accumulation of pro-inflammatory oxidized lipids in LDL using a cell-free assay developed on the basis of previously published method. Change in the fluorescence intensity of 2',7'-dichlorofluoresceine (DCFH) as a result of its oxidation by lipid peroxidation products, in the absence or presence of HDL, was used to monitor oxidation. Briefly, DCFH-diacetate (DCFH-DA) was dissolved in methanol at 2.0 mg/mL and incubated at room temperature for 30 minutes protected from light, resulting in the release of DCFH. Reference LDL isolated from one healthy normolipidemic subject (final concentration, 5 mg TC/dL) and AAPH (final concentration, 10 mM) were added to the DCFH-containing tubes following evaporation of methanol (final DCFH concentration, 2 mg/ml), followed by the addition of HDL (HDL subfractions, final concentration, 5 mg total mass/dL; total HDL, 20 mg total mass/dL). The volume was adjusted to 100 µl with Dulbecco's PBS and the reaction mixture transferred onto a microtiter plate. The plate was incubated at 37°C and the kinetics of fluorescence intensity measured over 24h with a fluorescence microplate reader (Spectra Max, Gemini XS; Molecular Devices, USA) at an excitation wavelength of 485 nm, emission wavelength of 530 nm, and cutoff of 515 nm.

### Anti-apoptotic activity

Anti-apoptotic activity of HDL was assessed as the capacity to protect endothelial cells from apoptosis. The human microvascular endothelial cell line, HMEC-1, was a kind gift of Dr Trottein (Institut Pasteur, Lille, France) and Dr Candal (CDC Atlanta, Atlanta, USA). This human microvascular endothelial cell line possesses biological characteristics similar to those of macrovascular endothelial cells in terms of mechanisms of toxicity induced by oxidized LDL (oxLDL), thereby representing an adequate model for macrocirculation. HMEC-1 were grown as described elsewhere and preincubated in the absence (control) or in the presence of each HDL subfraction (25 µg total protein/ml) in a serum-free RPMI medium for 24 h. Subsequently, oxLDL was added as to the final apoB concentration of 200 µg/ml and cells were further incubated for up to 24h. To prepare oxLDL, native LDL was fractionated by sequential ultracentrifugation from a plasma pool obtained from normolipidemic subjects at the Blood Transfusion Centre of the Hospital La Pitié-Salpetrière. Mildly oxLDL was prepared by UV irradiation in the presence of Cu2+ (5 µM) as described elsewhere. Circulating levels of total protein in each HDL subfraction normally range from 10 to 30 mg/dl; the working HDL concentration chosen by us corresponded to 4-12-fold dilutions. Since the concentration of apoB represents a 3-5-fold dilution vs. normal plasma levels, the resulting HDL/oxLDL ratio therefore adequately models the expected physiological ratio of HDL/oxLDL. To specifically induce primary apoptosis, mildly oxLDL containing controlled levels of LDL lipid peroxidation products (Δ234 nm, 0.050-0.150 corresponding to 4-13 mol of conjugated dienes/mol LDL) were used in all experiments. Such low levels of LDL oxidation correspond to approximately 5-10% of the theoretical maximum, thereby modeling typical plasma levels of oxidatively modified LDL which represent about 1-10% of the total LDL pool.

Cytotoxicity induced by oxLDL was evaluated using the 3-(4,5-dimethyl-2-yl)-2,5-diphenyltetrazolium bromide (MTT) assay, which measures mitochondrial dehydrogenase activity; 20 the assay was performed as described elsewhere. The predominance of cellular death via primary apoptosis rather than necrosis was confirmed by flow cytometry analysis using the Annexin V- FITC / propidium iodide kit as described elsewhere.

### Statistical analysis

All results are expressed as means +/-SD. Between-group differences were analyzed using the Student's t-test. Pearson's correlation coefficients were calculated to evaluate relationships between variables. Heat maps were constructed on the basis of correlation coefficients using a freely available R language, http://www.r-project.org.

### Lipidome of HDL particle subpopulations in healthy normolipidemic subjects

An original LC-MS/MS methodology for PL and sphingolipid (SL) profiling involving reverse-phase LC separation was applied to the analysis of human plasma HDL subpopulations isolated by isopycnic density gradient ultracentrifugation. This approach features separation of analytes and internal standards as a key step and allows matrix effects and varying ionization efficiencies to be accurately taken into account. For quantification, a set of non-naturally occurring internal standards were added prior to lipid extraction.

Using this methodology, 162 individual molecular lipid species were identified in five normolipidemic HDL subpopulations across the nine lipid subclasses, including 23 PC, 22 SM, 9 LPC, 25 PE, 17 PI, 11 PG, 24 ceramide (Cer), 18 PS and 13 PA species. PC species clearly predominated, accounting together for 73 to 77% of total PL+SL, followed by SM (15-21%), LPC (2.4-3.9%), PE (1.5-2.2%), PI (1.7-2.4%), PG (0.27-0.37%), Cer (0.09-0.16%), PS (0.03-0.53%) and PA (0.02-0.05%) species.

A high level of heterogeneity in lipid content was found across HDL subpopulations. While absolute levels of the majority of lipid subclasses in HDL followed circulating concentrations of HDL particles, SM and Cer tended to be enriched in large, light HDL, whereas PS preferentially associated with small, dense particles. As a result, when expressed as a wt% of PL+SL, PS, but also PC, LPC, and PA, showed a marked tendency to increase progressively in parallel with increase in hydrated density and reduction in size from HDL2b to HDL3c. Indeed, small, dense HDL were enriched in PC, PS and PA relative to large, light HDL (p=0.01, p=0.003 and p>0.001 for trend, respectively; **Fig. 1**). Furthermore, HDL3c was enriched in LPC (+48%, p=0.05) and PS (17-fold, p<0.01) relative to HDL2b (**Fig. 1**). Similarly, PE, PI and PG tended to concentrate in small, dense HDL; these trends did not however attain significance (**Fig. 1**). Interestingly, lipid species of PS were localized almost exclusively in the densest HDL3c subfractions; PS content varied from 0.03% of total PL+SL in HDL2b to 0.53% in HDL3c. As a consequence, the percentage of negatively charged PLs PI, PS, PG and PA increased with HDL density from 2.0% of total PL+SL in HDL2b to 3.3% in HDL3c. Very similar profiles of lipid subclasses across HDL subpopulations were observed when their content in HDL was expressed as molar %. In addition, the profiles of the major individual molecular species within each lipid subclass were similar across HDL particle subpopulations (data not shown).

Concomitant with such enrichment in PLs, the proportion of SM and Cer decreased progressively in parallel with HDL density from 20% and 0.16% of total PL+SL in HDL2b to 15% and 0.10% in HDL3c, respectively (**Fig. 1**). As a result, the SM/PC ratio decreased from 0.28 in HDL2b to 0.18 in HDL3c, consistent with earlier data. Similar relationships between HDL content of lipid subclasses and density were also observed when they were expressed on the basis of total HDL lipids.

Consistent with published data, HDL content of major lipid classes (PL, FC, CE, TG) calculated on the basis of total HDL mass showed a distinct trend to decrease with increment in total protein content and particle density across the HDL particle spectrum. In addition, HDL content of FC (calculated on the basis of total lipids) equally decreased from 9.4% in HDL2b to 5.3% in HDL3c, whereas PL, CE and TG did not reveal clear trends with HDL density.

### Biological activities of HDL particle subpopulations in healthy normolipidemic subjects

The capacity of individual HDL subpopulations to mediate cellular efflux of free cholesterol was evaluated in macrophage-like human THP-1 cells which efflux cholesterol predominantly via the ABCA1-dependent pathway. On the basis of unit PL mass content, both small, dense HDL3b and 3c particles displayed greater efficacy in removing cellular cholesterol as compared to other HDL subpopulations (p<0.001; **Fig. 2****, A**). Thus, the cholesterol efflux capacity varied from 4.48% for HDL2b to 8.02% for HDL3c. Moreover, HDL3c exhibited higher cholesterol efflux capacity than HDL3b (p<0.001).

Anti-oxidative activity of HDL particles was assessed as inhibition of free radical-induced LDL oxidation. Consistent with previous data, the inhibitory effects of small, dense HDL3b and HDL3c on LDL oxidation were superior relative to large, light HDL2b on the basis of total mass, with respect to both reduction in LDL oxidation rate in the propagation phase (3.8-fold, p<0.05, and 5.2-fold, p<0.01, respectively; **Fig. 2****, B**) and increases in the duration of this phase (+623%, p<0.01, and +697%, p<0.01, respectively; **Fig. 2****, C**).

Antithrombotic activity of HDL subpopulations was evaluated as their ability to inhibit H2O2-induced phosphorylation of p38-MAPK and thromboxane B2 production in human platelets on the basis of total protein content. Small, dense HDL3c prevented H2O2-induced phosphorylation of p38-MAPK to a greater degree than lighter HDL2b, 2a, 3a and 3b subpopulations (p<0.05 for all comparisons; **Fig. 2****, D**). Moreover, HDL3c inhibited thromboxane B2 production induced by hydrogen peroxide more efficiently than HDL2b (+300%, p<0.01; **Fig. 2****, E**).

Anti-inflammatory activity evaluated using a cell-free assay on a total mass basis tended to be predominantly associated with small, dense HDL. Indeed, HDL3b and 3c tended to attenuate generation of pro-inflammatory oxidized PLs to a greater degree as compared to other HDL subpopulations (**Fig. 2****, F**).

Finally and consistent with earlier data, the small, dense HDL3c subfraction potently protected human microvascular endothelial cell line, HMEC-1, from apoptotic death induced by oxidized LDL (**Fig. 2****, G**). On a total protein basis, HDL3c exhibited 2.0-fold superior anti-apoptotic activity relative to HDL2b (p < 0.05).

The biological activities of HDL particles were strongly intercorrelated. Furthermore, such activities exhibited significant correlations with multiple components of the HDL phosphosphingolipidome. Specifically, the content of phosphatidylserine revealed positive correlations with all metrics of HDL functionality, reflecting enrichment of PS in small, dense HDL3.

### Enhancement of anti-atherosclerotic activities of HDL by PS.

Inclusion of PS in apoA-I/PC-containing rHDL significantly enhanced cholesterol efflux capacity from THP-1 cells (**Fig. 3****, A**), anti-inflammatory activity towards LPS-activated macrophages (**Fig. 3****, C**), anti-oxidative activity towards LDL oxidation (**Fig. 3****, D**), anti-apoptotic activity in HMEC-1 (**Fig. 3****, E**) and anti-thrombotic activity in human platelets (**Fig. 3****, F**). In addition, in vitro enrichment of HDL3c improved cholesterol efflux capacity of human HDL3c; this effect was less pronounced as compared to that observed upon HDL3c enrichment in PC (**Fig. 3****, B**). Remarkably, LPS-induced activation of macrophages was completely blocked by PS-containing rHDL (**Fig. 3****, C**).

### Influence of other negatively charged PLs on anti-atherosclerotic activities of HDL.

In vitro enrichment of native human plasma HDL in PG was performed. To enrich HDL in PG, plasma (1.5 ml in the case of PI or 3 ml in the case of PG) was incubated in the presence of indicated amounts of PG and total HDL was isolated by density gradient ultracentrifugation. Control HDL was isolated from plasma incubated in the presence of PBS. Cholesterol efflux was assessed in lipid-loaded THP-1 cells and expressed as % [3H]-cholesterol efflux. HDL particles were compared on the basis of unit PL mass content. In vitro enrichment of native human plasma HDL in PG improved cholesterol efflux capacity of HDL from THP-1 cells (**Fig. 4**). On the contrary, enrichment of HDL in PA reduced both cholesterol efflux capacity and anti-oxidative activity of HDL (**Fig. 5**). To enrich HDL in PA, total plasma HDL (1.5 mg PL) was incubated in the presence of indicated amounts of PA and HDL was re-isolated by density gradient ultracentrifugation. As a control, HDL was isolated from plasma incubated in parallel with PBS. Cholesterol efflux was assessed in lipid-loaded THP-1 cells and expressed as % [3H]-cholesterol efflux. HDL particles were compared on the basis of unit PL mass content. Anti-oxidative activity of HDL was assessed on the basis of total mass content towards LDL oxidation and expressed as a decrease in the LDL oxidation rate in the propagation phase.

## Claims

1. A reconstituted high density lipoprotein composition comprising apolipoprotein, fragments or combinations thereof and at least one negatively charged phospholipid, fragments or combinations thereof.

2. The reconstituted high density lipoprotein composition according to claim **1,** wherein said reconstituted high density lipoprotein is a small, dense HDL particle.

3. The reconstituted high density lipoprotein composition according to anyone of claims **1** to **2,** wherein said small, dense HDL particle has a density between 1.12 and 1.25 g/mL, preferably 1.18 and 1.25 g/mL.

4. The reconstituted high density lipoprotein composition according to claim anyone of **1** to **3,** wherein said reconstituted high density lipoprotein has a size between 6 to 12 nm, preferably 8 to 10 nm, most preferably 8 to 9 nm.

5. The reconstituted high density lipoprotein composition according to anyone of claims **1** to **4,** wherein the negatively charged phospholipid is selected from the groups of: phosphatidyl serine, phosphatidyl inositol, phosphatidyl glycerol, most preferably phosphatidyl serine.

6. The reconstituted high density lipoprotein composition according to anyone of claims **1** to **5,** further comprising at least one zwitterionic phospholipid.

7. The reconstituted high density lipoprotein composition according to claim **6,** wherein said zwitterionic phospholipid is phosphatidyl choline (PC).

8. The reconstituted high density lipoprotein composition according to anyone of claims **1** to **7,** wherein said reconstituted high density lipoprotein further comprises a detergent.

9. The reconstituted high density lipoprotein composition according to anyone of claims **1** to **8,** wherein said reconstituted high density lipoprotein further comprises a stabilizer.

10. The reconstituted high density lipoprotein composition according to anyone of claims **1** to **9,** for use in the treatment of a disorder related to the dysfunction of HDL metabolism.

11. The reconstituted high density lipoprotein composition according to anyone of claims **1** to **9,** for use in the treatment of an inflammatory disorder.

12. The reconstituted high density lipoprotein composition according to anyone of claims **1** to **9,** for use in the treatment of a cardiovascular disorder.
